(19) Europäisches Patentamt
European Patent Office
Office européen des brevets

(11) EP 1 605 051 B1

(12) EUROPEAN PATENT SPECIFICATION

(45) Date of publication and mention of the grant of the patent:
**02.04.2014 Bulletin 2014/14**

(51) Int Cl.:
***C12N 15/82*** *(2006.01)*

(21) Application number: **05104022.8**

(22) Date of filing: **13.05.2005**

(54) **Method for increasing transgene expression**

Verfahren zur Erhöhung der Transgenexpression

Méthode pour augmenter l'expression d'un transgène

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **13.05.2004 EP 04102108**
**18.05.2004 US 572141 P**

(43) Date of publication of application:
**14.12.2005 Bulletin 2005/50**

(73) Proprietor: **CropDesign N.V.**
**9052 Zwijnaarde (BE)**

(72) Inventors:
- **Hatzfeld, Yves**
  **59000, Lille (FR)**
- **Broekaert, Willem**
  **1700, Dilbeek (BE)**
- **De Wilde, Chris**
  **9310, Herdrsem-Aalst (BE)**
- **Zhou, Zhongyi**
  **9860, Balegem (BE)**

(74) Representative: **Popp, Andreas**
**BASF SE**
**Global Intellectual Property**
**GVX/B - C 6**
**Carl-Bosch-Str. 38**
**67056 Ludwigshafen (DE)**

(56) References cited:
**WO-A-00/32800**

- **DATABASE EMBL [Online] 6 December 2000 (2000-12-06), "Binary vector pINDEX1, complete sequence." XP002308213 retrieved from EBI accession no. EM_PRO:AF294979 Database accession no. AF294979 -& OUWERKERK P.B. ET AL.: "Glucocorticoid-inducible gene expression in rice" PLANTA (BERLIN), vol. 213, no. 3, July 2001 (2001-07), pages 370-378, XP002308212 ISSN: 0032-0935**
- **MCELROY D. ET AL: "Construction of expression vectors based on the rice actin 1 (Act1) 5' region for use in monocot trransformation" MOL. GEN. GENET, XX, XX, vol. 231, 1991, pages 150-160, XP002118811**
- **MCELROY D. ET AL.: "Isolation of an efficient actin promoter for use in rice transformation" PLANT CELL, AMERICAN SOCIETY OF PLANT PHYSIOLOGISTS, ROCKVILLE, MD, US, vol. 2, no. 2, February 1990 (1990-02), pages 163-171, XP002118812 ISSN: 1040-4651**
- **HENSGENS L.A.M. ET AL.: "TRANSIENT AND STABLE EXPRESSION OF GUSA FUSIONS WITH RICE GENES IN RICE, BARLEY AND PERENNIAL RYEGRASS" PLANT MOLECULAR BIOLOGY, NIJHOFF PUBLISHERS, DORDRECHT, NL, vol. 23, 1993, pages 643-669, XP002925025 ISSN: 0167-4412**
- **DATABASE EMBL [Online] 21 February 1991 (1991-02-21), "O.sativa (rice) constitutive GOS2 gene" XP002308214 retrieved from EBI accession no. EM_PRO:OSGOS2G Database accession no. X51910 -& DE PATER B.S. ET AL.: "The promoter of the rice gene GOS2 is active in various different monocot tissues and binds rice nuclear factor ASF-1" PLANT J.., vol. 2, no. 6, 1992, pages 837-844, XP000907326**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 605 051 B1

- TANAKA A. ET AL: "Enhancement of foreign gene expression by a dicot intron in rice but not in tobacco is correlated with an increased level of mRNA and an efficient splicing of the intron" NUCLEIC ACIDS RESEARCH, OXFORD UNIVERSITY PRESS, SURREY, GB, vol. 18, no. 23, 11 December 1990 (1990-12-11), pages 6767-6770, XP002023309 ISSN: 0305-1048

## Description

The present invention relates to methods for increasing transgene expression in plants. In particular, the present invention relates to the use of the 5' untranslated region (5'UTR) of a GOS2 gene, particularly to the use of the first intron of a GOS2 gene for enhancing transcription. This invention also relates to methods for modifying the composition of seeds and in particular for increasing transcription or protein expression in plants.

Protein levels in a cell are determined on the one hand by their rate of degradation and on the other hand by their rate of synthesis. The rate of protein synthesis is dependent on various processes, such as transcription, post-transcriptional RNA processing, translation and post-translational modifications. The first step in protein synthesis is transcription, the process of copying DNA into an RNA molecule. Besides the promoter, which is the most important part of a gene controlling the level and pattern of transcription, it is also well known that the 5' untranslated region (5'UTR) of a primary RNA transcript has influence on the level and pattern of expression of plant genes. Several plant genes are known to contain an intron in their 5'UTR; gene expression and regulation studies have demonstrated that these introns may have a positive impact on transcription. Examples include introns from *Arabidopsis* (Rose and Last, Plant J. 11, 455-464, 1997; Chaubet-Gigot et al., Plant Mol. Biol. 45, 17-30, 2001), castor bean (*Ricinus communis*; Tanaka et al., Nucleic Acids Res. 18, 6767-6770, 1990), oat (*Avena sativa*; Bruce and Quail, Plant Cell 2, 1081-1089, 1990), petunia (*Petunia hybrida;* Dean et al., Plant Cell 1, 201-208, 1989; Vain et al., Plant Cell Rep. 15, 489-494, 1996), rice (*Oryza sativa*; McElroy et al., Plant Cell 2, 163-171, 1990; Snowden et al., Plant Mol. Biol. 31, 689-692, 1996; Rethmeier et al., Plant J. 12, 895-899, 1997), soybean (*Glycine max;* Kato et al., Biosci. Biotechnol. Biochem. 62, 151-153, 1998), potato (*Solanum tuberosum*; Leon et al., Plant Physiol. 95, 968-972, 1991; Fu et al., Plant Cell 7, 1387-1394, 1995; Fu et al., Plant Cell 7, 1395-1403, 1995), and tobacco (*Nicotiana tabacum;* Plesse et al., Plant Mol. Biol. 45, 655-667, 2001).

The mechanism underlying this intron-mediated enhancement of transcription is still unclear, but the introns must be in their normal orientation with respect to the transcribed region of the gene (Callis et al., Genes Dev. 1, 1183-1200, 1987; Clancy et al., Plant Sci. 98, 151-161, 1994; Mascarenhas et al., Plant Mol. Biol. 15, 913-920, 1990). A study of *PAT1* intron 1 in *Arabidopsis* (Rose, RNA 8, 1444-1453, 2002) demonstrated that the splicing machinery was required, but that intron splicing was not enough to enhance mRNA accumulation. According to the model presented, introns could stimulate mRNA accumulation by increasing the processing capacity of RNA polymerase II via a modification of its carboxy-terminal domain, thereby promoting transcript elongation without significantly affecting transcript initiation. The transcription of genes containing an intron would therefore be more likely to extend the length of the gene in cases where 3' end processing would produce stable transcripts. Studies suggest that the presence of an intron increases the probability of full-length stable transcripts being made, thereby leading to increased mRNA accumulation (Rose, 2002). Clancy and Hannah (Plant Physiol. 130, 918-929, 2002) used reporter gene fusions to identify features of the Sh1 first intron required for enhancement in cultured maize cells. They found that a 145-bp derivative of the Sh1 intron conferred approximately the same 20- to 50-fold stimulation typical for the full-length intron in their transient expression system. Furthermore, a 35-bp motif contained within the intron was found to be required for maximum levels of enhancement but not for efficient transcript splicing. The important feature of this redundant 35-bp motif is T-richness rather than the specific sequence. When transcript splicing was abolished by mutations at the intron borders, enhancement was reduced to about 2-fold. The requirement of splicing for enhancement was found not to be due to upstream translation initiation codons contained in unspliced transcripts. The authors concluded that splicing of the Sh1 intron was integral to enhancement and suggested that transcript modifications triggered by the T-rich motif and splicing may link the mRNA with the trafficking system of the cell. Other reports suggest that length and composition of flanking sequences may contribute to intron-mediated enhanced gene expression (Sinibaldi and Mettler, In WE Cohn, K Moldave, eds, Progress in Nucleic Acid Research and Molecular Biology, Vol. 42. Academic Press, New York, pp 229-257, 1992; Luehrsen and Walbot, Prog. Nucleic Acid Res. Mol. Biol. 47, 149-193, 1991; Maas et al., Plant Mol. Biol. 16, 199-207, 1991; Clancy et al., 1994), or that the magnitude of stimulation also depends on the coding sequences (Sinibaldi and Mettler, 1992; Rethmeier et al., 1997; Rethmeier et al., Plant J. 13, 831-835, 1998), the tissue of expression, and physiological conditions (Tanaka et al., 1990; Sinibaldi and Mettler, 1992; Gallie and Young, Plant Physiol. 106, 929-939, 1994; Fu and Park, 1995a, 1995b; Chaubet-Gigot et al., 2001; Plesse et al., 2001).

*GOS2* from rice is a single copy gene which encodes the translation initiation factor elF1, which is involved in the assembly of the 43S complex with mRNA and in the correct scanning of the 5'UTR towards the ATG during initiation of translation. The *GOS2* gene is also found in other organisms such as yeast and mammals, and for each of these, the first intron is located in the 5'UTR. In its natural environment, the rice GOS2 protein is expressed under control of its strong and constitutive promoter. This promoter is widely used as an alternative for the CaMV35S promoter in the genetic engineering of monocotyledonous plants. Hensgens et al. (Plant Mol. Biol. 23, 643-669, 1993) studied expression of the reporter gene *gusA* driven by the *GOS2* promoter. They describe a first type of construct comprising, from 5' to 3' a *GOS2* transcriptional promoter, the translation initiation site, the first two exons and introns and part of the third exon, a polylinker region, the *gusA* coding sequence and a polyadenylation region. Hensgens et al. also describe a second type of construct, in which *gusA* was fused to the first 36 nucleotides of the first *GOS2* exon and thus lacked the first two introns. The highest GUS expression was observed for the first type of construct, however the authors could not determine whether the deference in expression level between the two types of constructs was attributable to the presence of the two introns. To date, no analysis has been performed of the role of the introns in the GOS2 gene in the process of transcription.

Plant phenotypic properties are determined by expression of one or more genes in the plant genome. The appropriate expression level and pattern (temporal and spatial expression patterns) of genes may depend on many regulatory elements, including the type of promoter and the untranslated regions of the mRNA. These regulatory elements may advantageously be used in adapting expression of genes to particular needs. For example, a promoter may have the desired spatial expression (such as root-specific or flower-specific expression), but may lack the strength to ensure high expression levels. In such cases, a person skilled in the art may consider using additional regulatory elements to enhance expression levels. Examples of regulatory elements for modulating expression levels or patterns are known in the art and include upstream activating sequences, promoter-proximal elements, enhancers and silencers. A number of genes have been described for which the presence of introns contributed significantly to the expression level. However the mechanism of this increase is poorly understood.

In order to obtain desired gene expression levels or patterns, which may be fine-tuned depending on specific needs, it is desirable to have at one's disposal a wide array of introns that are capable of stimulating gene expression. A need exists in various fields of genetic engineering for transcriptional control elements capable of increasing gene expression in plants. The inventors have now shown for the first time that the 5' untranslated region (5'UTR), and particularly the first intron of a plant *GOS2* gene, has a stimulating effect on transgene expression in plants when inserted between a promoter and a nucleic acid to be expressed. This was found not only to be the case when the first intron of a plant *GOS2* gene was combined with the rice *GOS2* promoter, but was surprisingly also observed when a plant *GOS2* 5'UTR, and in particular its first intron, was operably linked to a plant-expressible non-*GOS2* promoter.

In one embodiment, the invention concerns a method for increasing transgene expression in a transgenic plant, which method comprises

a. integrating the first intron of the 5' UTR of a plant *GOS2* gene in or at the 5' end of a nucleic acid of interest, thereby creating a chimeric transcriptional unit, wherein said first intron is represented in SEQ ID NO: 5 or a functional variant of said first intron, which functional variant is at least 65 base pairs in length, comprises splice sites and a functional branchpoint adenosine, said branchpoint adenosine is part of the conserved motif with the following consensus sequence $Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4 x SSC at 65 °C, followed by a washing step in 0.1 x SSC at 65 °C for one hour;
b. operably fusing said chimeric transcriptional unit to a plant-expressible promoter so as to obtain an expression cassette, provided there is no combination of the 5' UTR as represented in SEQ ID NO: 2 from the rice GOS2 gene with a promoter of the rice *GOS2* gene;
c. introducing into and expressing in a plant cell said expression cassette of (b), to create a transgenic plant cell; and
d. regenerating and/or growing a plant from said transgenic plant cell of (c) so that said transgenic cell transcribes the transgene and wherein the 5' UTR of the expressed nucleic acid of interest comprises at least the first intron of the 5' UTR as defined in (a).

Described is a method for increasing transgene expression in a transgenic plant, which method comprises

a. integrating all or a part of a 5' UTR of a plant *GOS2* gene in or at the 5' end of a nucleic acid of interest, thereby creating a chimeric transcriptional unit, wherein said part of a 5'UTR comprises at least the first intron of said GOS2 gene or a functional variant of said first intron;
b. operably fusing the chimeric transcriptional unit to a plant expressible promoter so as to obtain an expression cassette, provided there is no combination of a complete 5' UTR from the rice *GOS2* gene with a promoter of the rice *GOS2* gene;
c. introducing into and expressing in a plant cell the expression cassette of (b), to create a transgenic plant cell; and
d. regenerating and/or growing a plant from the transgenic plant cell of (c) so that the transgenic cell transcribes the transgene and wherein the 5'UTR of the expressed nucleic acid of interest comprises at least the 5'UTR of (a).

The subject of the present invention concerns the first intron of a *GOS2* gene. The *GOS2* gene (coding sequence given in SEQ ID NO: 3), also known as *SUI1*, encodes a translation initiation factor eIF1 (SEQ ID NO: 4). In a particular embodiment, the first intron of the rice *GOS2* gene is used, but a person skilled in the art will appreciate that first introns of other *GOS2* genes may also be used in the methods of the invention. The rice *GOS2* gene contains four introns of respectively 998 bp, 94 bp, 90 bp and 105 bp (de Pater et al., Plant J. 2, 837-844, 1992). The first intron is located within the 5'UTR (Fig. 1), not only in rice (X51910), but also in *Coffea arabica* (AJ519840), and *Arabidopsis* (At5g54940, At5g54760, At1g54290, At4g27130). It may be expected that this is the case for all plants, given the high sequence conservation among plant *GOS2* genes. Therefore, in a preferred embodiment of this invention, alternative intron sequences of *GOS2* genes are derived from plant *GOS2* genes, because in these genes, the first introns are located in the 5'UTR. More preferably, the first intron of a *GOS2* gene is derived from a monocotyledonous plant, most preferably from rice or maize. Nevertheless, a person skilled in the art would be able to isolate *GOS2* first intron sequences from other plants without undesired flanking coding sequences by using standard techniques. Therefore, first introns derived from any plant *GOS2*/*SUI1* gene may equally be useful in the methods of this invention.

The term "intron" or "intervening sequence" as defined herein is a non-coding nucleic acid sequence frequently interrupting coding sequences of eukaryotic genes but which may also be present outside the coding sequence and which are transcribed into RNA and subsequently removed by RNA splicing to leave a functional mRNA or other RNA. The intron comprises splice sites at both ends and a branchpoint adenosine. The term "splice sites" as defined herein is taken to mean the outer ends of the sequence that are spliced out from the primary transcript and the immediate nucleotides in the primary transcript of the sequence that is spliced out. The branchpoint adenosine is the nucleotide that forms the lariat during the event of splicing. The branchpoint adenosine is part of a conserved motif with the following consensus sequence (Brown, Plant Journal 10, 771-780, 1996): $Y_{100}U_{100}R_{64}A_{100}U_{50}$ or $Y_{100}U_{100}R_{64}A_{100}Y_{70}$, wherein Y stands for the nucleotides C or T, and R for A or G and wherein the subscript numbers denote the percentage of conservation of the respective nucleotide. This motif determines the functionality of the branchpoint adenosine.

Furthermore, functional variants of the first intron of a *GOS2* gene may also be useful in increasing expression of a transgene. Functional variants encompass substitution, insertion and deletion variants and combinations thereof. "Substitution variants" are those in which at least one base in the nucleotide sequence has been removed and a different base inserted in its place. "Insertional variants" of a nucleic acid are those in which one or more nucleotides are introduced into a predetermined site in the sequence. "Deletion variants" of a nucleic acid are characterised by the removal of one or more nucleotides from the nucleic acid. Any combination of substitution(s), deletion(s) or insertion(s) may occur provided that the functionality of the intron remains essentially the same, that is, that the intron variant, when used in the methods according to the present invention, causes increased expression of a transgene. The increased expression is to be understood as increased when compared to expression of the same transgene without the presence of the intron. In addition, a functional variant is at least 65 base pairs in length, comprises splice sites and a functional branchpoint adenosine, and is able to hybridise under stringent conditions to a naturally occurring first intron of a plant GOS2 gene.

The term "hybridisation" as defined herein is a process wherein substantially homologous complementary nucleotide sequences anneal to each other. In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration and hybridisation buffer composition. "Stringent hybridisation conditions" and "stringent hybridisation wash conditions" in the context of nucleic acid hybridisation experiments such as Southern and Northern hybridisations are sequence dependent and are different under different environmental parameters. For example, longer sequences hybridise specifically at higher temperatures. The Tm is the temperature under defined ionic strength and pH, at which 50% of the target sequence hybridises to a perfectly matched probe. Specificity is typically the function of post-hybridisation washes. Critical factors of such washes include the ionic strength and temperature of the final wash solution. Generally, stringent conditions are selected to be about 50°C lower than the thermal melting point ($T_m$) for the specific sequence at a defined ionic strength and pH. The $T_m$ is the temperature under defined ionic strength and pH, at which 50% of the target sequence hybridises to a perfectly matched probe. The $T_m$ is dependent upon the solution conditions and the base composition of the probe, and may be calculated using the following equation:

$$T_m = 79.8°C + (18.5 x \log[Na^+]) + (58.4°C x \%[G+C]) - (820 x (\#bp\ in\ duplex)^{-1}) - (0.5 x \%\ formamide)$$

Preferred stringent conditions are when the temperature is 20°C below $T_m$, and most preferred stringent conditions are when the temperature is 10°C below $T_m$. Non-specific binding may also be controlled using any one of a number of known techniques such as blocking the membrane with protein containing solutions, additions of heterologous RNA, DNA, and SDS to the hybridization buffer, and treatment with RNase. Wash conditions are typically performed at or below hybridisation stringency. Generally, suitable stringent conditions for nucleic acid hybridisation assays or gene amplification detection procedures are as set forth above. Conditions of increased or decreased stringency compared to the above may also be selected.

For the purposes of defining the level of stringency, reference can conveniently be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989). The skilled artisan is aware of various parameters which may be altered during hybridisation and washing and which will either maintain or change the stringency conditions. An example of high stringency conditions includes hybridisation in 0.1-1x SSC / 0.1% w/v SDS at 60°C for 1-3 hours. Another example of stringent hybridisation conditions is hybridisation at 4x SSC at 65°C, followed by a washing in 0.1x SSC, at 65°C for about one hour. Alternatively, an exemplary stringent hybridisation condition is in 50% formamide, 4x SSC at 42°C. Still another example of stringent conditions include hybridisation at 62°C in 6x SSC, 0.05x BLOTTO and washing at 2x SSC, 0.1 % w/v SDS at 62°C.

Plant *GOS2* genes have their first intron located within the 5'UTR, therefore it is envisaged that it is not only this first intron but also the first intron with its flanking sequences up to the whole of the 5'UTR that may have a stimulating effect on gene expression. Therefore, the present invention provides in another embodiment a method for increasing transgene expression in a transgenic plant wherein substantially the complete 5'UTR of a plant *GOS2* gene, or any part thereof comprising the first intron or a functional variant thereof, is used to increase expression of a transgene, provided that the rice *GOS2* promoter is not used in combination with the complete 5' UTR of the rice *GOS2* gene.

The 5'UTR of a gene is to be understood as that part of a gene which is transcribed into a primary RNA transcript (pre-mRNA) and which part is located upstream of the coding sequence. The primary transcript is the initial RNA product, containing introns and exons, produced by transcription of DNA. Many primary transcripts must undergo RNA processing to form the physiologically active RNA species. The processing into a mature mRNA may comprise trimming of the ends, removal of introns, capping and/or cutting out of individual rRNA molecules from their precursor RNAs. The 5'UTR of an mRNA is thus that part of the mRNA which is not translated into protein and which is located upstream of the coding sequence.

According to the present invention, the 5'UTR of a GOS2 gene or a part thereof comprising the first intron, is integrated in or at the 5' end of a nucleic acid of interest. The fusion results in the formation of a transcriptional unit, which gives rise to a single functional primary RNA transcript that can be processed to a mature mRNA in a given host cell. The 5'UTR of a GOS2 gene, or a part thereof comprising the first intron, is present in the transcriptional unit in sense orientation and upstream of any protein encoding sequence. The intron useful in the methods of the present invention is usually flanked by the border sequences which comprise the splice site, but may also be functional without these border sequences.

Preferably the nucleic acid of interest is a transgene. The term "nucleic acid sequence(s)", "nucleic acid ", or "gene(s)", when used herein refers to nucleotides, either ribonucleotides (RNA) or deoxyribonucleotides (DNA) or a combination of both, in a polymeric form of any length. These terms furthermore include double-stranded and single-stranded DNA and RNA. These terms also include nucleotide modifications known in the art, such as methylation, cyclisation, 'caps', substitution of one or more of the naturally occurring nucleotides with an analogue such as inosine, and polynucleotide backbone modifications. The term "transgene" refers to an isolated nucleic acid that is introduced into a plant. In a preferred embodiment of the present invention, the transgene encodes a polypeptide.

The term "polypeptide" or "protein" is defined as a molecular chain comprising two or more amino acids covalently joined by peptide bonds.

The term "isolated" in the present application means removed from its original environment. For example, a nucleic acid present in its natural state in an organism is not isolated, whereas the same nucleic acid separated from the adjacent nucleic acids in which it is naturally present, is regarded as being "isolated". The transgene may originate from the same plant species or from another, it may be isolated from any source, such as bacteria, yeast or fungi, plants (including algae) or animals (including humans). The transgene may be substantially modified from its native form in composition and/or genomic environment through deliberate human manipulation. In a particular embodiment of the invention, the transgene comprises a sequence encoding a protein. However the transgene need not encode a protein, the methods may be applied for any nucleic acid to be expressed, such as tRNA, rRNA, anti-sense RNA, siRNA. A "coding sequence" or "open reading frame" (ORF) is defined as a nucleotide sequence that may be transcribed into mRNA and may later be translated into a polypeptide when placed under the control of appropriate regulatory sequences, i.e. when the coding sequence or ORF is present in an expressible form. The coding sequence or ORF is bound by a 5' translation start codon and a 3' translation stop codon. A coding sequence or ORF may include, but is not limited to RNA, mRNA, cDNA, recombinant nucleotide sequences, synthetically manufactured nucleotide sequences or genomic DNA. The coding sequence or ORF may be interrupted by introns.

Advantageously the methods of the present invention result in increased expression of the transgene. The term "expression" as defined herein is taken to mean the production of a protein or nucleotide sequence in a cell itself or in a cell-free system. It includes transcription into an RNA product, post-transcriptional modification and optionally also translation to a protein product or polypeptide from a DNA encoding that product, as well as possible post-translational modifications. A coding sequence may be transcribed in sense or antisense direction.

As mentioned above, any *GOS2*/*SUI1* gene may be a suitable source of a 5'UTR and/or a first intron for use in a method for increasing transgene expression. Methods for the search and identification of eIF1 homologues would be well within the realm of persons skilled in the art. Such methods comprise comparison of the sequences represented by SEQ ID NO 3 or 4, in a computer readable format, with sequences that are available in public databases such as MIPS (http://mips.gsf.de/), GenBank (http://www.ncbi.nlm.nih.gov/Genbank/index.html) or EMBL Nucleotide Sequence Database (http://www.ebi.ac.uk/embl/index.html), using algorithms well known in the art for the alignment or comparison of sequences, such as GAP (Needleman and Wunsch, J. Mol. Biol. 48; 443-453 (1970)), BESTFIT (using the local homology algorithm of Smith and Waterman (Advances in Applied Mathematics 2; 482-489 (1981))), BLAST (Altschul, S.F., Gish, W., Miller, W., Myers, E.W. & Lipman, D.J., J. Mol. Biol. 215:403-410 (1990)), FASTA and TFASTA (W. R. Pearson and D. J. Lipman Proc.Natl.Acad.Sci. USA 85:2444- 2448 (1988)). The software for performing BLAST analysis is publicly available through the National Centre for Biotechnology Information. Suitable homologues may be identified using BLAST default parameters (BLOSUM62 matrix, gap opening penalty 11 and gap extension penalty 1). Preferably, full-length sequences (from start to stop codon in case of DNA, or of the complete mature protein) are used for analysis.

Analysis of genomic sequences for the identification of *GOS2*/*SUI1* homologues is also possible. Several algorithms and software tools for the identification of genes in raw DNA sequence are available. Usually these tools combine analysis of statistical parameters in the DNA sequence with homology-based methods for identifying homologous sequences in databases. Although none of these methods alone is reliable enough for a good prediction, the combination of various programs usually gives good results. Well known examples of such tools include GeneMark (http://opal.biology.gatech.edu/genemark, http://www.ebi.ac.uk/genemark, Borodovsky, M. and McIninch J. (1993) GeneMark: Parallel Gene Recognition for both DNA Strands. Computers & Chemistry, 17, 123-133), Gene Locator and Interpolated Markov Modeler (GLIMMER, http://www.tigr.org/softiab, A.L. Delcher et al. Improved microbial gene identification with GLIMMER. Nucleic Acids Research, 27, 4636-4641.(1999)), Gene Recognition and Assembly Internet Link (GRAIL, http://compbio.ornl.gov), GenScan (http://genes.mit.edu/GENSCAN.html, Burge, C. and Karlin, S. (1997) Prediction of complete gene structures in human genomic DNA. J. Mol. Biol. 268, 78-94.), GeneBuilder (http://www.itba.mi.cnr.it/webgene Milanesi L. et al. GeneBuilder: interactive in silico prediction of genes structure. Bioinformatics, 15 (7):612-621, 1999). A combined analysis may be performed with the TIGR Combiner program (http://www.tigr.org/softlab, J. E. Allen et al. Computational gene prediction using multiple sources of evidence. Genome Research, 14(1), 142-148, 2004.) that predicts gene models using the output from other annotation software such as GeneMark, GlimmerM, GRAIL, GenScan, and Fgenes. It uses a statistical algorithm to identify patterns of evidence corresponding to gene models.

Methods for characterising the 5'UTR of a gene are known in the art. For example, many transcription starting points in eukaryotes are preceded by a TATA box or consist of an initiator element recognised by RNA polymerase II. Naturally occurring initiator elements in many genes have a cytosine (C) at the -1 position and an adenine (A) residue at the transcription-start site (+1). The consensus sequence for an initiator element consists of 5'-Y Y $A^{+1}$ N T/A Y Y Y-3', where $A^{+1}$ is the transcription start, Y is a pyrimidine (C or T), N is any of the four bases, and T/A is T or A at position +3. Other genes contain a typical CG-rich stretch of 20 to 50 nucleotides within a region of approximately 100 base pairs upstream of the start-site region, known as CpG island. Methods for predicting the presence of introns are also known in the art. Examples include SPL (http://www.softberry.com/berry.phtml , Softberry), NNSplice (http://www.fruitfly.org/seq_tools/splice.html, Reese MG et al. 1997. Improved Splice Site Detection in Genie. J Comp Biol 4(3), 311-23), SpliceView (http://www.itba.mi.cnr.it/webgene/, Rogozin I.B. and L. Milanesi. Analysis of donor splice signals in different organisms. J. Mol. Evol., 1997, V.45, 50-59.), NetGene2 (http://www.cbs.dtu.dk/services, S.M. Hebsgaard, et al. Splice site prediction in Arabidopsis thaliana DNA by combining local and global sequence information, Nucleic Acids Research, 1996, Vol. 24, 3439-3452.), GeneSplicer (http://www.tigr.org/softlab, Pertea M. et al. GeneSplicer: a new compulational method for splice site prediction. Nucleic Acids Res. 2001 Mar 1;29(5):1185-90).

In a preferred embodiment, the present invention provides a method for increasing expression of a transgene as outlined above, wherein said part of the GOS2 5'UTR is as represented by SEQ ID NO 1. In an alternative embodiment, the complete GOS2 5'UTR as represented by SEQ ID NO 2 is used. In any case, the methods according to the present invention always make use of a chimeric transcriptional unit comprising the nucleic acid of interest fused at its 5' end to the 3' end of the 5'UTR of a GOS2 gene, or part thereof, wherein that part of the GOS2 5'UTR comprises at least the first intron of the GOS2 gene or a functional variant of this first intron. Preferably, the part of the GOS2 5'UTR is as presented by SEQ ID NO 1.

As is generally accepted, a nucleic acid strand is directional, with the "5' end" having a free hydroxyl (or phosphate) on a 5' carbon and the "3' end" having a free hydroxyl (or phosphate) on a 3' carbon. In case of a double stranded nucleic acid, the orientation of the sense strand establishes the direction. The sense strand has, by definition, the same 'sense' as the mRNA; that is, it may be translated exactly as the mRNA sequence can.

In a next step, the transcriptional unit is operably linked to a promoter, thereby forming an expression cassette. As used herein, a "promoter" means a region of DNA upstream from the transcription start and which is involved in binding RNA polymerase and other proteins to start transcription. Reference herein to a "promoter" is to be taken in its broadest context and includes the transcriptional regulatory sequences derived from a classical eukaryotic genomic gene, including the TATA box which is required for accurate transcription initiation, with or without a CCAAT box sequence and additional regulatory elements (i.e. upstream activating sequences, enhancers and silencers) which alter gene expression in response to developmental and/or external stimuli, or in a tissue-specific manner. Consequently, a repressible promoter's rate of transcription decreases in response to a repressing agent. An inducible promoter's rate of transcription increases in response to an inducing agent. A constitutive promoter's rate of transcription is not specifically regulated, though it may vary under the influence of general metabolic conditions. The term "promoter" also includes the transcriptional regulatory sequences of a classical prokaryotic gene, in which case it may include a -35 box sequence and/or a -10 box transcriptional regulatory sequences. The term "promoter" is also used to describe a synthetic or fusion molecule, or derivative which confers, activates or enhances expression of a nucleic acid molecule in a cell, tissue or organ.

Advantageously, any type of promoter may be combined with the transcriptional unit and used to drive expression of the transgene, depending on the desired outcome. Advantageously, the promoter is a plant expressible promoter. By "plant expressible promoter" is meant a promoter which is capable of functioning in a plant cell. For example, a meristem-specific promoter, such as the rnr (ribonucleotide reductase), cdc2a promoter and the cyc07 promoter, may be used to effect expression in all growing parts of the plant, thereby increasing cell proliferation, which in turn would increase yield or biomass. If the desired outcome would be to influence seed characteristics, such as the storage capacity, seed size, seed number, biomass etc., then a seed-specific promoter, such as p2S2, pPROLAMIN, pOLEOSIN may be selected. An aleurone-specific promoter may be selected in order to increase growth at the moment of germination, thereby increasing the transport of sugars to the embryo. An inflorescence-specific promoter, such as pLEAFY, may be utilised if the desired outcome would be to modify the number of flower organs. To produce male-sterile plants one might want to use an anther specific promoter. To impact on flower architecture, for example petal size, one may choose a petal-specific promoter. If the desired outcome would be to modify growth and/or developmental characteristics in particular organs, the choice of the promoter to combine with would depend on the organ to be modified. For example, use of a root-specific promoter would lead to increased growth and/or increased biomass or yield of the root and/or phenotypic alteration of the root. This would be particularly important where it is the root itself that is the desired end product; such crops include sugar beet, turnip, carrot, and potato. A fruit-specific promoter may be used to modify, for example, the strength of the outer skin of the fruit or to increase the size of the fruit. A green tissue-specific promoter may be used to increase leaf size. A cell wall-specific promoter may be used to increase the rigidity of the cell wall, thereby increasing pathogen resistance. An anther-specific promoter may be used to produce male-sterile plants. A vascular-specific promoter may be used to increase transport from leaves to seeds. A nodule-specific promoter may be used to increase the nitrogen fixing capabilities of a plant, thereby increasing the nutrient levels in a plant. A stress-inducible promoter may also be used to drive expression of a nucleic acid to increase membrane integrity during conditions of stress. A stress-inducible promoter such as the water stress-inducible promoter WSI18, the drought stress-inducible Trg-31 promoter, the ABA related promoter rab21 or any other promoter which is inducible under a particular stress condition such as temperature stress (cold, freezing, heat) or osmotic stress, or drought stress or oxidative stress or biotic stress may be used to drive expression of a transgene.

In one particular embodiment of the invention, the promoter used for increasing the expression of a transgene is a constitutive promoter. The term "constitutive" as defined herein refers to a promoter expressed predominantly in at least one tissue or organ and predominantly at any life stage of the plant. Preferably the promoter is expressed predominantly throughout the plant. Preferably, the constitutive promoter is a *GOS2* promoter, or a promoter of similar strength and/or a promoter with a similar expression pattern, more preferably the promoter is the *GOS2* promoter from rice, provided that the rice *GOS2* promoter is not used in combination with the complete 5' UTR of the rice *GOS2* gene. In another embodiment, an inducible promoter is used. An inducible promoter's rate of transcription increases in response to an inducing agent or condition, such as chemical, environmental, or physical stimuli. In still another embodiment, a developmentally regulated promoter is used. The term "developmentally regulated promoter" refers to a promoter whose activity is determined by developmental events. In a preferred embodiment, an organ-preferred promoter is used. An organ-preferred promoter is a promoter that is predominantly expressed in a specific organ of the plant. In a more preferred embodiment, a seed-preferred promoter is used. The term seed-preferred promoter refers to a promoter that is predominantly expressed in seeds of a plant. In another preferred embodiment, a tissue-preferred promoter is used. The term "tissue-preferred" as defined herein refers to a promoter that is expressed predominantly but not necessarily exclusively in one tissue or organ, but that may also be expressed in one specific cell. Preferably the tissue-preferred promoter is an endosperm-preferred promoter. Further preferably, the endosperm preferred promoter is a prolamin RP6 promoter, more preferably the prolamin RP6 promoter from rice.

The inventors have also shown for the first time that a 5'UTR originating from a plant GOS2 gene or a part thereof, and which part comprises the first intron, can be used to increase expression of a transgene in plant seeds.

Therefore, there is provided a method for increasing protein content of plant seeds, which method comprises

a. integrating the first intron of the 5' UTR of a plant *GOS2* gene in or at the 5' end of a nucleic acid encoding a protein of interest, thereby creating a chimeric transcriptional unit, wherein said first intron is represented in SEQ ID NO: 5 or a functional variant of said first intron, which functional variant is at least65 base pairs in length, comprises splice sites and a functional branchpoint adenosine, said branchpoint adenosine is part of the conserved motif with the following consensus sequence $Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4 x SSC at 65 °C, followed by a washing step in 0.1 x SSC at 65 °C for one hour;
b. operably fusing said chimeric transcriptional unit to a plant-expressible promoter so as to obtain an expression cassette, provided there is no combination of the 5' UTR as represented in SEQ ID NO: 2 from the rice *GOS2* gene with a promoter of the rice *GOS2* gene;
c. introducing into and expressing in a plant cell the expression cassette of (b) to create a transgenic plant cell; and
d. regenerating and/or growing a plant from the transgenic plant cell of (c) so that said transgenic plant cell transcribes said nucleic acid encoding the protein of interest and wherein the 5' UTR of the expressed nucleic acid encoding said protein comprises at least the first intron of the 5' UTR of (a).

Described is a method for increasing protein content of plant seeds, which method comprises

a. integrating all or a part of the 5' UTR of a plant *GOS2* gene in or at the 5' end of a nucleic acid encoding the protein of interest, thereby creating a chimeric transcriptional unit, wherein this part of a *GOS2* 5'UTR comprises at least the first intron of the *GOS2* gene or a functional variant of this first intron;
b. operably fusing the chimeric transcriptional unit to a plant expressible promoter so as to obtain an expression cassette, provided there is no combination of a complete 5' UTR from the rice *GOS2* gene with a promoter of the rice *GOS2* gene;
c. introducing into and expressing in a plant cell the expression cassette of (b) to create a transgenic plant cell; and
d. regenerating and/or growing a plant from the transgenic plant cell of (c) so that the transgenic cell transcribes the nucleic acid encoding the protein of interest and wherein the 5'UTR of the expressed nucleic acid encoding the protein of interest comprises at least the 5'UTR of (a).

The term "operably linked" refers to a juxtaposition between a regulatory and a coding sequence, wherein the components so described are in a relationship permitting them to function in their intended manner. A regulatory sequence "operably linked" to a coding sequence is ligated in such a way that expression of the coding sequence is achieved under conditions compatible with the control sequences. In the case where the regulatory sequence is a promoter, it would be known to a skilled person that a double-stranded nucleic acid is preferable.
According to a further embodiment, there is provided a vector in which the expression cassette may be ligated. With "vector" or "genetic construct" is meant a DNA sequence, which may be introduced in an organism by transformation and may be transiently or stably maintained in this organism. Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences other than the first intron of a GOS2 gene, which may be suitable for use in performing the invention. Such sequences would be known or may readily be obtained by a person skilled in the art. The vector may further include an origin of replication sequence which is required for maintenance and/or replication in a specific cell type. One example is when a vector is required to be maintained in a bacterial cell as an episomal genetic element (e.g. plasmid or cosmid molecule). Preferred origins of replication include, but are not limited to, the f1-ori and colE1. The genetic construct may optionally comprise a selectable marker gene. As used herein, the term "selectable marker gene" includes any gene which confers a phenotype to a cell in which it is expressed to facilitate the identification and/or selection of cells which are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as *nptII* that phosphorylates neomycin and kanamycin, or *hpt,* phosphorylating hygromycin); genes conferring resistance to herbicides (for example *bar* which provides resistance to Basta; *aroA* or *gox* providing resistance against glyphosate); or genes that provide a metabolic trait (such as *manA* that allows plants to use mannose as sole carbon source). Visual marker genes result in the formation of colour (for example β-glucuronidase, *GUS*), luminescence (such as luciferase) or fluorescence (Green Fluorescent Protein, *GFP,* and derivatives thereof).
In a next step, the expression cassette, whether or not inserted in a vector, is introduced into a plant cell or into the plant itself (including introduction into a tissue, organ or any other part of the plant). According to a preferred feature of the present invention, the nucleic acid is preferably introduced into a plant by transformation. The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom.

The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome or into the chloroplast genome. The resulting transformed plant cell may then be used to regenerate a transformed plant in a manner known to persons skilled in the art.

Transformation of a plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., 1982, Nature 296, 72-74; Negrutiu I. et al., 1987, Plant Mol. Biol. 8, 363-373); electroporation of protoplasts (Shillito R.D. et al., 1985, Bio/Technol. 3, 1099-1102); microinjection into plant material (Crossway A. et al., 1986, Mol. Gen. Genet. 202, 179-185); DNA or RNA-coated particle bombardment (Klein T.M. et al., 1987, Nature 327, 70) infection with (non-integrative) viruses and the like. A preferred method for rice transformation according to the present invention is via *Agrobacterium*-mediated transformation using any of the well known methods for rice transformation, such as described in any of the following: published European patent application EP 1198985 A1, Aldemita and Hodges (Planta, 199, 612-617, 1996); Chan et al. (Plant Mol. Biol. 22 (3) 491-506, 1993), Hiei et al. (Plant J. 6 (2) 271-282, 1994). In the case of corn transformation, the preferred method is as described in either Ishida et al. (Nat. Biotechnol. 1996 Jun; 14(6): 745-50) or Frame et al. (Plant Physiol. 2002 May; 129(1): 13-22).

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis or by PCR, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

In a next step of selection, transformed plants are evaluated for the desired expression of the transgene or for the desired phenotypes. Methods for measuring modulated expression (increased or decreased expression) are known to the person skilled in the art. Altered RNA levels may be measured with techniques like RT-PCR whereas changes in protein levels may be determined by enzymatic assays, staining of SDS-PAGE protein gels, Western blotting, or enzyme-linked immunosorbent assays. Furthermore, in case of transgenic plants, changes in the phenotype of the transgenic plants may be monitored. For example, changes in yield or biomass, changes in plant architecture or stress response, or changes in protein or oil content of seeds may be measured. It is known to persons skilled in the art that the expression of transgenes in plants, and hence also the phenotypic effect due to expression of such transgene, may differ among different independently obtained transgenic lines and progeny thereof. The transgenes present in different independently obtained transgenic plants may differ from each other by the chromosomal insertion locus as well as by the number of transgene copies inserted, and by the configuration of those multiple transgene copies in a locus. Therefore differences in expression levels may be expected, which may be due to influence from the chromosomal context of the transgene (the so-called position effect) or from silencing mechanisms triggered by certain transgene configurations (e.g. inwards facing tandem insertions of transgenes are prone to silencing at the transcriptional or post-transcriptional level).

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods according to this invention. The invention also extends to harvestable parts of a plant, such as (but not limited to) seeds, leaves, fruits, flowers, stems or stem cultures, rhizomes, roots, tubers and bulbs. The invention further relates to products derived directly from a harvestable part of such a plant, such products including dry pellets or powders, oil, fat and fatty acids, starch or proteins.

The term "plant" as used herein encompasses whole plants, ancestors and progeny of the plants and plant parts, including seeds, fruits, flowers, leaves, shoots, stems, roots (including tubers), and plant cells, tissues and organs. The term "plant" therefore also encompasses suspension cultures, embryos, meristematic regions, callus tissue, gametophytes, sporophytes, pollen, and microspores. Plants that are particularly useful in the methods of the invention include algae, ferns, and all plants which belong to the superfamily Viridiplantae, in particular monocotyledonous and dicotyledonous plants, including fodder or forage legumes, ornamental plants, food crops, trees, or shrubs selected from the list comprising *Abelmoschus* spp., *Acer* spp., *Actinidia* spp., *Agropyron* spp., *Allium* spp., *Amaranthus* spp., *Ananas comosus, Annona* spp., *Apium graveolens*, *Arabidopsis thaliana, Arachis* spp, *Artocarpus* spp., *Asparagus officinalis*, *Avena sativa*, *Averrhoa carambola, Benincasa hispida*, *Bertholletia excelsea, Beta vulgaris*, *Brassica* spp., *Cadaba farinosa*, *Camellia sinensis, Canna indica*, *Capsicum* spp., *Carica papaya, Carissa macrocarpa*, *Carthamus tinctorius, Carya* spp., *Castanea* spp., *Cichorium endivia*, *Cinnamomum spp., Citrullus lanatus, Citrus* spp., *Cocos* spp., *Coffea* spp., *Cola* spp., *Colocasia esculenta, Corylus* spp., *Crataegus* spp., *Cucumis* spp., *Cucurbita* spp., *Cynara* spp., *Daucus carota, Desmodium* spp., *Dimocarpus longan, Dioscorea* spp., *Diospyros* spp., *Echinochloa* spp., *Eleusine coracana*, *Eriobotrya japonica, Eugenia uniflora, Fagopyrum* spp., *Fagus* spp., *Ficus carica, Fortunella spp.*, *Fragaria* spp., *Ginkgo biloba*, *Glycine* spp., *Gossypium hirsutum, Helianthus* spp., *Hibiscus* spp., *Hordeum* spp., *Ipomoea batatas*, *Juglans* spp., *Lactuca sativa*, *Lathyrus* spp., *Lemna spp., Lens culinaris, Linum usitatissimum*, *Litchi chinensis, Lotus* spp., *Luffa acutangula*, *Lupinus* spp., *Macrotyloma* spp., *Malpighia emarginata, Malus* spp., *Mammea americana*, *Mangifera indica, Manihot* spp., *Manilkara* zapota, *Medicago sativa, Melilotus* spp., *Mentha* spp., *Momordica* spp., *Morus nigra, Musa* spp., *Nicotiana* spp., *Olea* spp., *Opuntia* spp., *Omithopus* spp., *Oryza* spp., *Panicum miliaceum*, *Passiflora edulis*, *Pastinaca sativa, Persea* spp., *Petroselinum crispum*, *Phaseolus* spp., *Phoenix* spp., *Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis*, *Quercus* spp., *Raphanus sativus*, *Rheum rhabarbarum, Ribes* spp., *Rubus* spp., *Saccharum* spp., *Sambucus* spp., *Secale cereale, Sesamum* spp., *Solanum* spp., *Sorghum bicolor, Spinacia* spp., *Syzygium* spp., *Tamarindus indica*, *Theobroma cacao*, *Trifolium* spp., *Triticosecale rimpaui*, *Triticum* spp., *Vaccinium* spp., *Vicia* spp., *Vigna spp.*, *Vitis* spp., *Zea mays, Zizania palustris, Ziziphus* spp., amongst others.

According to a preferred feature of the present invention, the plant is a crop plant comprising soybean, sunflower, canola, alfalfa, rapeseed or cotton. Further preferably, the plant according to the present invention is a monocotyledonous plant, including members of the Poaceae, such as sugarcane; most preferably a cereal, such as rice, maize, wheat, millet, barley, oats, sorghum.

The present invention also describes the use of the 5'UTR of a plant *GOS2* gene or part thereof comprising the first intron. Any use of the 5'UTR of a plant *GOS2* gene or part thereof comprising the first intron, for increasing transgene expression is envisaged, provided there is no combination of a complete 5' UTR from the rice *GOS2* gene with a promoter of the rice *GOS2* gene. In particular, the 5'UTR of a plant *GOS2* gene or part thereof comprising the first intron may be used for modifying the growth characteristics of a plant, such as yield, amount of produced biomass, architecture, stress tolerance. Other examples of use of the 5'UTR of a plant *GOS2* gene or part thereof comprising the first intron include modification of the composition of a plant or harvestable parts thereof, such as seeds. The invention relates to the use of the first intron which is represented in SEQ ID NO: 5 or a functional variant of said first intron, which functional variant is at least 65 base pairs in length, comprises splice sites and a functional branchpoint adenosine, said branchpoint adenosine is part of the conserved motif with the following consensus sequence $Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4 x SSC at 65 °C, followed by a washing step in 0.1 x SSC at 65 °C for one hour in a method for modifying the composition of seeds of a plant, provided there is no combination of the 5' UTR as represented in SEQ ID NO: 2 from the rice *GOS2* gene with a promoter of the rice *GOS2* gene. Such use may result for example in seeds with increased protein content. The invention relates to the use of the first intron which is represented in SEQ ID NO: 5 or a functional variant of said first intron, which functional variant is at least 65 base pairs in length, comprises splice sites and a functional branchpoint adenosine, said branchpoint adenosine is part of the conserved motif with the following consensus sequence $Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4 x SSC at 65 °C, followed by a washing step in 0.1 x SSC at 65 °C for one hour in a method for increasing protein content of plant seeds, provided there is no combination of the 5' UTR as represented in SEQ ID NO: 2 from the rice *GOS2* gene with a promoter of the rice *GOS2* gene. Increased protein content of seeds is advantageous for increasing the nutritional value of these seeds or is furthermore advantageous in the field of molecular farming. Plants with improved growth characteristics may also be used to produce industrial proteins and/or other compounds. The invention relates to the use of the first intron which is represented in SEQ ID NO: 5 or a functional variant of said first intron, which functional variant is at least 65 base pairs in length, comprises splice sites and a functional branchpoint adenosine, said branchpoint adenosine is part of the conserved motif with the following consensus sequence

$Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4 x SSC at 65 °C, followed by a washing step in 0.1 x SSC at 65°C for one hour in a method for

(i) increasing the expression of a nucleic acid of interest fused thereto;
(ii) modifying growth characteristics of a plant;
(iii) increasing yield of a plant;
(iv) increasing stress tolerance of a plant; or
(v) altering architecture of a plant,

provided there is no combination of the 5' UTR as represented in SEQ ID NO: 2 from the rice *GOS2* gene with the promoter of the rice *GOS2* gene. In this case, the goal would be to ensure high accumulation of the desired products in particular and easy-to-harvest plant tissues.

The present invention also describes a composition comprising the 5'UTR of a plant GOS2 gene, or part thereof, for increasing expression of a transgene in transgenic plants provided that (i) said part of the GOS2 5'UTR comprises at least the first intron of said GOS2 gene or a functional variant of said first intron and (ii) the rice GOS2 intron is not used in combination with rice GOS2 promoter.

The present invention will now be described with reference to the following figures in which:

**Figure 1**: Schematic representation of the rice *GOS2* gene. The bottom arrow shows the structure of the mRNA, the white parts represent the introns, whereas the top arrow depicts the corresponding coding sequence (filled parts).

**Figure 2**: Schematic representation of the T-DNA of plasmid p5024, comprising starting from the left border (LB) a *nptII* expression cassette for resistance to kanamycin (SM k7) within 2 recombination sites (RS), the promoter of the rice prolamin *RP6* gene, the cocksfoot mottle virus (CfMV) epsilon leader (enhancer, GenBank Z48630), the Gateway cassette A (GW), containing a chloramphenicol resistance gene and a *ccdB* suicide gene flanked by Gateway recombination site *attR1* and *attR2*, a double terminator sequence (*T-zein* and *T-rbcS*) flanked by the right border (RB) sequence of the T-DNA.

**Figure 3:** Schematic representation of the T-DNA of plasmid p5942, derived from p5024 by inserting the first intron of rice GOS2 in the *Spe*I restriction site.

**Figure 4:** Schematic representation of the T-DNA of plasmids p06192 and p06193, used for transforming rice plants. The GW cassette of p5942 and p5024 is replaced by a reporter gene.

**Figure 5:** DNA sequences useful in the present invention. SEQ ID NO 1 represents the part of the 5'UTR of rice *GOS2* which is used in the examples. The intron sequence itself (SEQ ID NO: 5) is indicated in bold. SEQ ID NO 2 represents the complete 5'UTR of the rice *GOS2* gene defined by de Pater et al. (1992), extracted from GenBank accession X51910. Transcription may start at any of the first three nucleotides. SEQ ID NO: 3 represents the coding sequence of the GOS2 translation initiation factor and SEQ ID NO: 4 is the GOS2 protein sequence encoded by SEQ ID NO: 3.

## Examples:

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Genetic constructs*

A modified T-DNA (p5024) was created comprising within its borders the elements as shown in Figure 2. Next the first intron of the rice *GOS2* gene was inserted in the restriction site *Spe*I (Figure 3). Finally, the GW cassette was replaced by the *GUS* gene using a GW LR reaction, resulting in the plasmids p06192 and p06193 (Figure 4).

***Example 2: Rice transformation:***

Mature dry seeds of *Oryza sativa* japonica cultivar Nipponbare were dehusked. Sterilization was done by incubating the seeds for one minute in 70% ethanol, followed by 30 minutes in 0.2% $HgCl_2$ and by 6 washes of 15 minutes with sterile distilled water. The sterile seeds were then germinated on a medium containing 2,4-D (callus induction medium). After a 4-week incubation in the dark, embryogenic, scutellum-derived calli were excised and propagated on the same medium. Two weeks later, the calli were multiplied or propagated by subculture on the same medium for another 2 weeks. Three days before co-cultivation, embryogenic callus pieces were sub-cultured on fresh medium to boost cell division activity. The *Agrobacterium* strain LBA4404 harbouring the binary vector p06192 or p06193 was used for co-cultivation. The *Agrobacterium* strain was cultured for 3 days at 28°C on AB medium with the appropriate antibiotics. The bacteria were then collected and suspended in liquid co-cultivation medium at an $OD_{600}$ of about 1. The suspension was transferred to a petri dish and the calli were immersed in the suspension for 15 minutes. The callus tissues were then blotted dry on a filter paper, transferred to solidified co-cultivation medium and incubated for 3 days in the dark at 25°C. Thereafter, co-cultivated callus was grown on 2,4-D-containing medium for 4 weeks in the dark at 28°C in the presence of a selective agent at a suitable concentration. During this period, rapidly growing resistant callus islands developed. Upon transfer of this material to a regeneration medium, and incubation in the light, the embryogenic potential was released and shoots developed in the next four to five weeks. Shoots were excised from the callus and incubated for 2 to 3 weeks on an auxin-containing medium from which they were transferred to soil. Hardened shoots were grown under high humidity and short days in a greenhouse. Finally seeds were harvested three to five months after transplanting. The method yielded Single locus transformants at a rate of over 50 % (Aldemita and Hodges, Planta 199; 612-617, 1996; Chan et al., Plant Mol. Biol. 22(3), 491-506, 1993; Hiei et al., Plant J. 6(2), 271-282, 1994).

***Example 3: evaluation of the transformed plants:***

Approximately 15 to 20 independent T0 transformants were generated. The primary transformants were transferred from tissue culture chambers to a greenhouse for growing and harvest of T1 seed. 10 and 15 events (respectively without and with intron) were retained. For each event, 10 T1 seeds (a 1:2:1 mix of nullizygotes, hemizygotes and homozygotes) were ground to a fine powder and assayed for GUS activity (modified from Breyne P, et al. (1993), Plant Mol. Biol. Reporter 11, 21-31) in triplicate measurements. Results are given in Table 1 below:

**Table 1**: Measurement of GUS activities in plants transformed with construct with or without GOS2 intron:

| **Promoter + Enhancer + GUS** | | | | | | **Promoter + GOS2-intron + Enhancer + GUS** | | | | |
|---|---|---|---|---|---|---|---|---|---|---|
| **Line** | **1** | **2** | **3** | **Average** | | **Line** | **1** | **2** | **3** | **Average** |
| OS1861-001A | 14.3 | 28.7 | 17.3 | 20 | | OS1866-001C | 67.1 | 109.5 | 80.4 | 86 |
| OS1861-001B | 8.8 | 11.1 | 11.4 | 10 | | OS1866-002C | 40.6 | 153.2 | 259.8 | 151 |
| OS1861-002A | 46.8 | 54.1 | 14.8 | 39 | | OS1866-003C | 158.5 | 129.6 | 200.1 | 163 |
| OS1861-004A | 28.4 | 14.7 | 24.7 | 23 | | OS1866-005A | 173.8 | 302.8 | 61.9 | 180 |
| OS1861-005C | 7.5 | 17.3 | 29.8 | 18 | | OS1866-005B | 162.1 | 217.1 | 65.6 | 148 |
| OS1861-006A | 13.4 | 53.2 | 14.5 | 27 | | OS1866-007A | 27.4 | 221.2 | 281.7 | 177 |
| OS1861-006B | 4.1 | 15.5 | 26.3 | 15 | | OS1866-007C | 72.4 | 130.2 | 183.8 | 129 |
| OS1861-007B | 2.5 | 106.4 | NA | 54 | | OS1866-008A | 196.6 | 186.2 | 117 | 167 |
| OS1861-009A | 18.3 | 118.7 | 92.4 | 76 | | OS1866-008C | 161.5 | 336.6 | 61.2 | 186 |
| OS1861-009C | 13.5 | 11.7 | 1.9 | 9 | | OS1866-011A | 162.0 | 238.1 | 403.4 | 268 |
| | | | **average** | **29** | | OS1866-012A | 118.7 | 115.4 | 449.8 | 228 |
| | | | stdev | 22 | | OS1866-013A | 33.6 | 55.4 | NA | 45 |
| | | | | | | OS1866-014A | 17.7 | 99.8 | 79 | 66 |
| | | | | | | OS1866-014B | 93.7 | 81.2 | 105.2 | 93 |

(continued)

| **Promoter + Enhancer + GUS** | | | | | **Promoter + GOS2-intron + Enhancer + GUS** | | | | |
|---|---|---|---|---|---|---|---|---|---|
| **Line** | **1** | **2** | **3** | **Average** | **Line** | **1** | **2** | **3** | **Average** |
| | | | | | OS1866-015A | 159.4 | 90.0 | 10.8 | 87 |
| | | | | | | | | **average** | **149** |
| | | | | | | | | stdev | 62 |

From the data it can be deduced that the presence of the GOS2 intron results in a more than 5-fold increase in expression of the reporter gene.

## Claims

1. Method for increasing transgene expression in a transgenic plant, which method comprises

    a) integrating the first intron of the 5' UTR of a plant *GOS2* gene in or at the 5' end of a nucleic acid of interest, thereby creating a chimeric transcriptional unit, wherein said first intron is represented in SEQ ID NO: 5 or a functional variant of said first intron, which functional variant is at least 65 base pairs in length, comprises splice sites and a functional branchpoint adenosine, said branchpoint adenosine is part of the conserved motif with the following consensus sequence $Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4x SSC at 65°C, followed by a washing step in 0.1x SSC at 65°C for one hour;
    b) operably fusing said chimeric transcriptional unit to a plant-expressible promoter so as to obtain an expression cassette, provided there is no combination of the 5' UTR as represented in SEQ ID NO: 2 from the rice GOS2 gene with a promoter of the rice GOS2 gene;
    c) introducing into and expressing in a plant cell said expression cassette of (b), to create a transgenic plant cell; and
    d) regenerating and/or growing a plant from said transgenic plant cell of (c) so that said transgenic cell transcribes the transgene and wherein the 5'UTR of the expressed nucleic acid of interest comprises at least the first intron of the 5'UTR as defined in (a).

2. Method of claim 1, wherein said transgene encodes a polypeptide.

3. Method according to claims 1 and 2, wherein said transgenic plant is a monocotyledonous plant.

4. Method of claim 3, wherein said monocotyledonous plant is a cereal, such as rice or maize.

5. Method of any one of claims 1 to 4 wherein said plant GOS2 gene originates from a monocotyledonous plant, preferably from rice or maize.

6. Method according to any one of claims 1 to 5, wherein said plant-expressible promoter is selected from the group consisting of:

    (i) a constitutive promoter;
    (ii) an inducible promoter;
    (iii) a tissue-preferred promoter;
    (iv) an organ-preferred promoter;
    (v) a developmentally regulated promoter.

**7.** Method of claim 6, wherein said organ-preferred promoter is a seed-preferred promoter.

**8.** Method of claim 6, wherein said tissue-preferred promoter is an endosperm-preferred promoter.

**9.** Method according to any one of claims 1 to 8, wherein said chimeric transcriptional unit comprises a nucleic acid of interest fused at its 5' end to the 3' end of the first intron of the 5'UTR of a plant GOS2 gene.

**10.** Use of first intron as represented, in SEQ ID NO: 5 or a functional variant of said first intron, which functional variant is at least 65 base pairs in length, comprises splice sites and a functional branchpoint adenosine, said branchpoint adenosine is part of the conserved motif with the following consensus sequence $Y_{100}U_{100}R_{64}A_{100}U_{50}$ or $Y_{100}U_{100}R_{64}A_{100}Y_{70}$, wherein Y stands for the nucleotides C or T, and R for A and G, and wherein the subscript numbers denote the percentage of conservation of the respective nucleotide, which functional variant is able to hybridize under stringent conditions to the first intron as represented in SEQ ID NO: 5, wherein said hybridization conditions are hybridization at 4x SSC at 65°C, followed by a washing step in 0.1 x SSC at 65°C for one hour in a method for

(i) increasing the expression of a nucleic acid of interest fused thereto;
(ii) modifying growth characteristics of a plant;
(iii) increasing yield of a plant;
(iv) increasing stress tolerance of a plant;
(v) altering architecture of a plant,
(vi) modifying the composition of seeds of a plant; or
(vii) increasing the protein content of plant seeds,

provided there is no combination of the 5' UTR as represented in SEQ ID NO: 2 from the rice *GOS2* gene with the promoter of the rice *GOS2* gene.

**Patentansprüche**

**1.** Verfahren zum Erhöhen der Transgenexpression in einer transgenen Pflanze, das Folgendes umfasst:

a) Integrieren des ersten Introns der 5'-UTR eines pflanzlichen *GOS2*-Gens in oder am 5'-Ende einer interessierenden Nukleinsäure, wodurch eine chimäre Transkriptionseinheit entsteht, wobei das erste Intron in SEQ. ID. NO: 5 dargestellt ist, oder einer funktionellen Variante des ersten Introns, die mindestens 65 Basenpaare lang ist, Spleißstellen und ein funktionelles Verzweigungspunkt-Adenosin umfasst, wobei das Verzweigungspunkt-Adenosin Bestandteil des konservierten Motivs mit der folgenden Konsensussequenz $Y_{100}U_{100}R_{64}A_{100}U_{50}$ oder $Y_{100}U_{100}R_{64}A_{100}Y_{70}$ ist, wobei Y für die Nukleotide C oder T und R für A oder G steht, und wobei die tiefgestellten Zahlen den Konservierungsprozentsatz der jeweiligen Nukleotide bedeuten, wobei die funktionelle Variante fähig ist, unter stringenten Bedingungen mit dem ersten Intron wie in SEQ. ID. NO: 5 dargestellt zu hybridisieren, wobei die Hybridisierungsbedingungen Hybridisierung bei 4x SSC bei 65°C, gefolgt von einem einstündigen Waschschritt in 0,1 x SSC bei 65°C, lauten;
b) operatives Verknüpfen der chimären Transkriptionseinheit mit einem in Pflanzen exprimierbaren Promoter, wodurch man zu einer Expressionskassette gelangt, mit der Maßgabe, dass keine Kombination der 5'-UTR gemäß SEQ. ID. NO: 2 aus dem Reis-*GOS2*-Gen mit einem Promoter des Reis-*GOS2*-Gens vorliegt;
c) Einführen und Exprimieren der Expressionskassette von (b) in eine(r) Pflanzenzelle, wodurch eine transgene Pflanzenzelle entsteht; und
d) Regenerieren und/oder Heranziehen einer Pflanze aus der transgenen Pflanzenzelle von (c), so dass die transgene Zelle das Transgen transkribiert und wobei die 5'-UTR der exprimierten interessierenden Nukleinsäure zumindest das erste Intron der 5'-UTR wie in (a) definiert umfasst.

**2.** Verfahren nach Anspruch 1, wobei das Transgen für ein Polypeptid codiert.

**3.** Verfahren nach den Ansprüchen 1 und 2, wobei es sich bei der transgenen Pflanze um eine monokotyle Pflanze handelt.

**4.** Verfahren nach Anspruch 3, wobei es sich bei der monokotylen Pflanze um ein Getreide, wie Reis oder Mais, handelt.

5. Verfahren nach einem der Ansprüche 1 bis 4, wobei das pflanzliche *GOS2*-Gen von einer monokotylen Pflanze, vorzugsweise von Reis oder Mais, stammt.

6. Verfahren nach einem der Ansprüche 1 bis 5, wobei der in Pflanzen exprimierbare Promoter aus der Gruppe bestehend aus den folgenden ausgewählt ist:

   (i) einem konstitutiven Promoter;
   (ii) einem induzierbaren Promoter;
   (iii) einem gewebebevorzugten Promoter;
   (iv) einem organbevorzugten Promoter;
   (v) einem entwicklungsregulierten Promoter.

7. Verfahren nach Anspruch 6, wobei es sich bei dem organbevorzugten Promoter um einen samenbevorzugten Promoter handelt.

8. Verfahren nach Anspruch 6, wobei es sich bei dem gewebebevorzugten Promoter um einen endospermbevorzugten Promoter handelt.

9. Verfahren nach einem der Ansprüche 1 bis 8, wobei die chimäre Transkriptionseinheit eine interessierende Nukleinsäure umfasst, die an ihrem 5'-Ende mit dem 3'-Ende des ersten Introns der 5'-UTR eines pflanzlichen *GOS2*-Gens fusioniert ist.

10. Verwendung des ersten Introns gemäß SEQ. ID. NO:5 oder einer funktionellen Variante des ersten Introns, die mindestens 65 Basenpaare lang ist, Spleißstellen und ein funktionelles Verzweigungspunkt-Adenosin umfasst, wobei das Verzweigungspunkt-Adenosin Bestandteil des konservierten Motivs mit der folgenden Konsensussequenz $Y_{100}U_{100}R_{64}A_{100}U_{50}$ oder $Y_{100}U_{100}R_{64}A_{100}Y_{70}$ ist, wobei Y für die Nukleotide C oder T und R für A oder G steht, und wobei die tiefgestellten Zahlen den Konservierungsprozentsatz der jeweiligen Nukleotide bedeuten, wobei die funktionelle Variante fähig ist, unter stringenten Bedingungen mit dem ersten Intron wie in SEQ. ID. NO: 5 dargestellt zu hybridisieren, wobei die Hybridisierungsbedingungen Hybridisierung bei 4x SSC bei 65°C, gefolgt von einem einstündigen Waschschritt in 0,1 x SSC bei 65°C, lauten, in einem Verfahren zum

    (i) Erhöhen der Expression einer damit fusionierten interessierenden Nukleinsäure;
    (ii) Modifizieren von Wachstumseigenschaften einer Pflanze;
    (iii) Erhöhen des Ertrags einer Pflanze;
    (iv) Erhöhen der Stresstoleranz einer Pflanze;
    (v) Verändern der Architektur einer Pflanze;
    (vi) Modifizieren der Zusammensetzung von Samen einer Pflanze; oder
    (vii) Erhöhen des Proteingehalts von Pflanzensamen,

    mit der Maßgabe, dass keine Kombination der 5'-UTR gemäß SEQ. ID. NO: 2 aus dem Reis-*GOS2*-Gen mit dem Promoter des Reis-*GOS2*-Gens vorliegt.

## Revendications

1. Procédé pour augmenter l'expression de transgène dans une plante transgénique, ledit procédé comprenant

   a) l'intégration du premier intron du 5'UTR d'un gène GOS2 de plante dans ou à l'extrémité 5' d'un acide nucléique d'intérêt, de manière à créer une unité transcriptionnelle chimérique, ledit premier intron étant représenté dans SEQ ID NO: 5 ou un variant fonctionnel dudit premier intron, ledit variant fonctionnel ayant une longueur d'au moins 65 paires de bases, comprend des sites d'épissage et un adénosine de point de branchement fonctionnelle, ladite adénosine de point de branchement fait partie du motif conservé ayant la séquence consensus suivante $Y_{100}U_{100}R_{64}A_{100}U_{50}$ ou $Y_{100}U_{100}R_{64}A_{100}Y_{70}$, où Y désigne les nucléotides C ou T, et R pour A et G, et où les nombres en indice désignent le pourcentage de conservation du nucléotide respectif, ledit variant fonctionnel étant capable de s'hybrider dans des conditions stringentes au premier intron tel que représenté dans SEQ ID NO: 5, lesdites conditions d'hybridation étant une hybridation à 4 x SSC à 65 °C, suivie d'une étape de lavage dans 0,1 x SSC à 65 °C pendant une heure ;
   b) la fusion opérationnelle de ladite unité transcriptionnelle chimérique à un promoteur exprimable dans une plante de manière à obtenir une cassette d'expression, à condition qu'il n'y ait pas de combinaison du 5'UTR tel que représenté dans SEQ ID NO: 2 du gène GOS2 de riz avec un promoteur du gène GOS2 de riz ;
c) l'introduction et l'expression dans une cellule de plante de ladite cassette d'expression de (b), pour créer une cellule de plante transgénique ; et
d) la régénération et/ou la croissance d'une plante à partir de ladite cellule de plante transgénique de (c) de sorte que ladite cellule transgénique transcrive le transgène et le 5'UTR de l'acide nucléique d'intérêt exprimé comprend au moins le premier intron du 5'UTR tel que défini dans (a).

**2.** Procédé de la revendication 1, dans lequel ledit transgène code pour un polypeptide.

**3.** Procédé selon les revendications 1 et 2, dans lequel ladite plante transgénique est une plante monocotylédone.

**4.** Procédé de la revendication 3, dans lequel ladite plante monocotylédone est une céréale, telle que le riz ou le maïs.

**5.** Procédé de l'une quelconque des revendications 1 à 4 dans lequel ledit gène GOS2 de plante provient d'une plante monocotylédone, de préférence du riz ou du maïs.

**6.** Procédé selon l'une quelconque des revendications 1 à 5, dans lequel ledit promoteur exprimable par une plante est choisi dans le groupe constitué de :

(i) un promoteur constitutif ;
(ii) un promoteur inductible ;
(iii) un promoteur préférentiel dans un tissu ;
(iv) un promoteur préférentiel dans un organe ;
(v) un promoteur régulé par le développement.

**7.** Procédé de la revendication 6, dans lequel ledit promoteur préférentiel dans un organe est un promoteur préférentiel dans la graine.

**8.** Procédé de la revendication 6, dans lequel ledit promoteur préférentiel dans un tissu est un promoteur préférentiel dans l'endosperme.

**9.** Procédé selon l'une quelconque des revendications 1 à 8, dans lequel ladite unité transcriptionnelle chimérique comprend un acide nucléique d'intérêt fusionné à son extrémité 5' à l'extrémité 3' du premier intron du 5'UTR d'un gène GOS2 de plante.

**10.** Utilisation du premier intron tel que représenté dans SEQ ID NO: 5 ou un variant fonctionnel dudit premier intron, ledit variant fonctionnel ayant une longueur d'au moins 65 paires de bases, comprend des sites d'épissage et une adénosine de point de branchement fonctionnelle, ladite adénosine de point de branchement fait partie du motif conservé ayant la séquence consensus suivante $Y_{100}U_{100}R_{64}A_{100}U_{50}$ ou $Y_{100}U_{100}R_{64}A_{100}Y_{70}$, où Y désigne les nucléotides C ou T, et R pour A et G, et où les nombres en indice désignent le pourcentage de conservation du nucléotide respectif, ledit variant fonctionnel étant capable de s'hybrider dans des conditions stringentes au premier intron tel que représenté dans SEQ ID NO: 5, lesdites conditions d'hybridation étant une hybridation à 4 x SSC à 65 °C, suivie d'une étape de lavage dans 0,1 x SSC à 65 °C pendant une heure dans un procédé pour

(i) augmenter l'expression d'un acide nucléique d'intérêt fusionnné à celui-ci ;
(ii) modifier les caractéristiques de croissance d'une plante ;
(iii) augmenter le rendement d'une plante ;
(iv) augmenter la tolérance au stress d'une plante ;
(v) modifier l'architecture d'une plante,
(vi) modifier la composition de graines d'une plante ; ou
(vii) augmenter la teneur en protéine de graines de plante,

à condition qu'il n'y ait pas de combinaison du 5'UTR tel que représenté dans SEQ ID N0: 2 du gène GOS2 de riz avec le promoteur du gène GOS2 de riz.

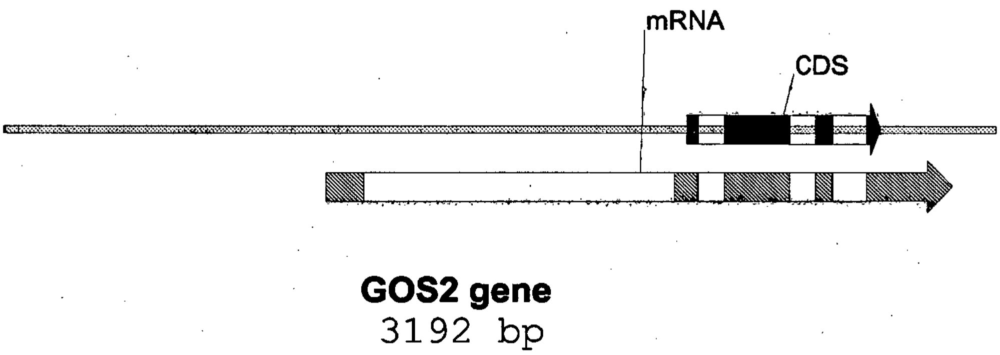


FIGURE 1

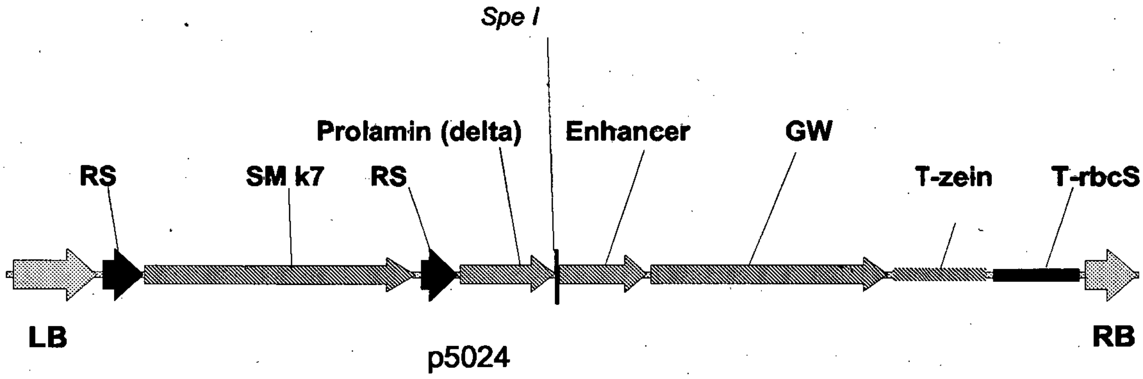


FIGURE 2

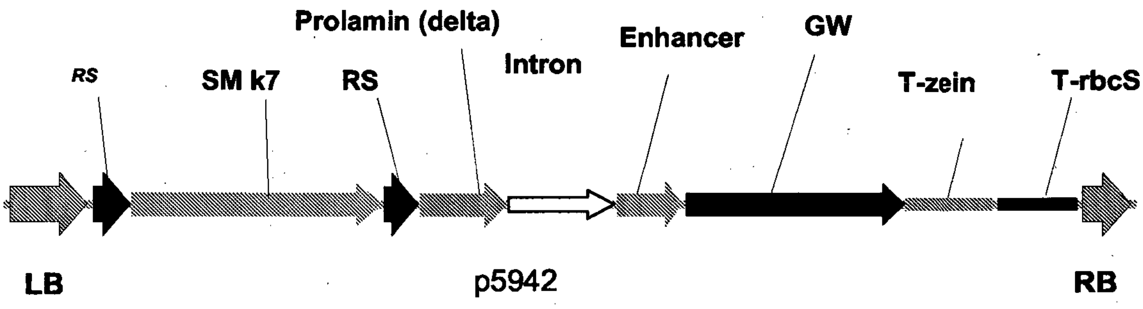


FIGURE 3

A

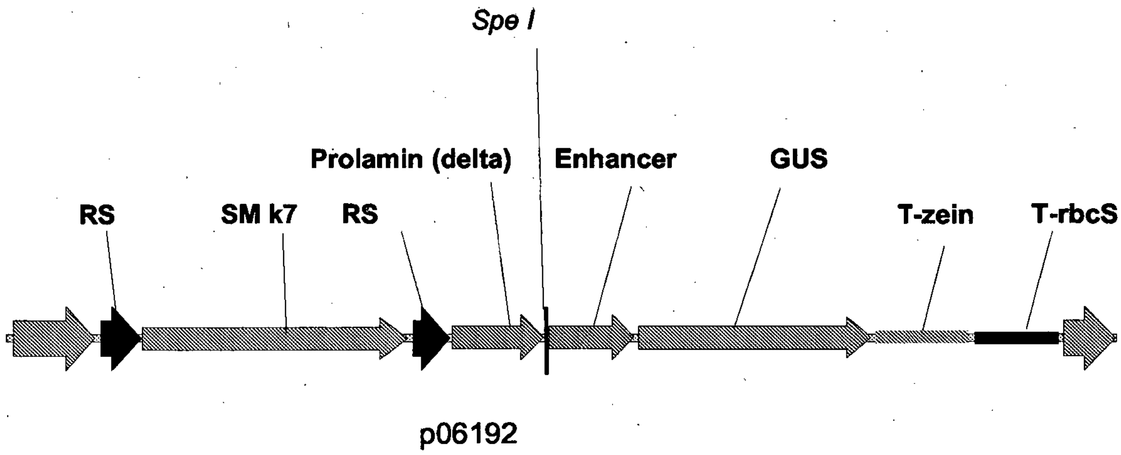
Spe I
Prolamin (delta)
Enhancer
GUS
RS
SM k7
RS
T-zein
T-rbcS
p06192

B

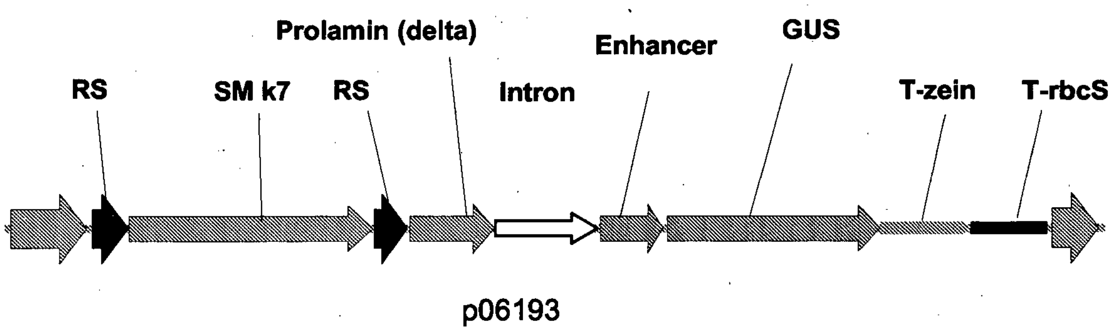
Prolamin (delta)
Enhancer
GUS
RS
SM k7
RS
Intron
T-zein
T-rbcS
p06193

FIGURE 4

**SEQ ID NO 1:**

ctagtagaagccgagcgaccgccttcttcgatccatatcttccggtcgagttcttggtcgat
ctcttccctcctccacctcctcctcacagg**gtatgtgcccttcggttgttcttggatttatt**
**gttctaggttgtgtagtacgggcgttgatgttaggaaaggggatctgtatctgtgatgattc**
**ctgttcttggatttgggatagaggggttcttgatgttgcatgttatcggttcggtttgatta**
**gtagtatggttttcaatcgtctggagagctctatggaaatgaaatggtttagggtacggaat**
**cttgcgattttgtgagtaccttttgtttgaggtaaaatcagagcaccggtgattttgcttgg**
**tgtaataaaagtacggttgtttggtcctcgattctggtagtgatgcttctcgatttgacgaa**
**gctatcctttgtttattccctattgaacaaaaataatccaactttgaagacggtcccgttga**
**tgagattgaatgattgattcttaagcctgtccaaaatttcgcagctggcttgtttagataca**
**gtagtccccatcacgaaattcatggaaacagttataatcctcaggaacaggggattccctgt**
**tcttccgatttgctttagtcccagaatttttttcccaaatatcttaaaagtcactttctg**
**gttcagttcaatgaattgattgctacaaataatgcttttatagcgttatcctagctgtagtt**
**cagttaataggtaataccccctatagtttagtcaggagaagaacttatccgatttctgatctc**
**catttttaattatatgaaatgaactgtagcataagcagtattcatttggattatttttttta**
**ttagctctcaccccttcattattctgagctgaaagtctggcatgaactgtcctcaattttgt**
**tttcaaattcacatcgattatctatgcattatcctcttgtatctacctgtagaagtttcttt**
**ttggttattccttgactgcttgattacagaaagaaatttatgaagctgtaatcgggatagtt**
**atactgcttgttcttatgattcatttcctttgtgcag**ttcttggtgtagcttgccact

**SEQ ID NO 2:**

taggaggcatccaagccaagaagagggagagcaccaaggacacgcgactagcagaagccgag
cgaccgccttcttcgatccatatcttccggtcgagttcttggtcgatctcttccctcctcca
cctcctcctcacagggtatgtgcccttcggttgttcttggatttattgttctaggttgtgta
gtacgggcgttgatgttaggaaaggggatctgtatctgtgatgattcctgttcttggatttg
ggatagaggggttcttgatgttgcatgttatcggttcggtttgattagtagtatggttttca
atcgtctggagagctctatggaaatgaaatggtttagggtacggaatcttgcgattttgtga
gtaccttttgtttgaggtaaaatcagagcaccggtgattttgcttggtgtaataaaagtacg
gttgtttggtcctcgattctggtagtgatgcttctcgatttgacgaagctatcctttgttta
ttccctattgaacaaaaataatccaactttgaagacggtcccgttgatgagattgaatgatt
gattcttaagcctgtccaaaatttcgcagctggcttgtttagatacagtagtccccatcacg
aaattcatggaaacagttataatcctcaggaacaggggattccctgttcttccgatttgctt
tagtcccagaatttttttcccaaatatcttaaaagtcactttctggttcagttcaatgaa
ttgattgctacaaataatgcttttatagcgttatcctagctgtagttcagttaataggtaat
acccctatagtttagtcaggagaagaacttatccgatttctgatctccatttttaattatat
gaaatgaactgtagcataagcagtattcatttggattatttttttttattagctctcacccct
tcattattctgagctgaaagtctggcatgaactgtcctcaattttgttttcaaattcacatc
gattatctatcgattatcctcttgtatctacctgtagaagtttctttttggttattccttga
ctgcttgattacagaaagaaatttatgaagctgtaatcgggatagttatactgcttgttctt
atgattcatttcctttgtgcagttcttggtgtagcttgccactttcaccagcaaagtttc

**FIGURE 5**

**SEQ ID NO 3:**

atgtctgatctcgacattcagatcccaactgccttcgatccctttgctgaggccaatgctgg
agactctggtgcggctgcaggatcaaaggactacgttcatgtacgcatccagcagcgtaatg
gccgtaagagcctgaccactgtccagggattgaagaaggaattcagctacaacaagatcctt
aaagatctcaagaaagagttttgctgcaatggtactgttgtccaggacccagagcttggcca
ggtcattcaacttcagggtgatcagaggaagaacgtatcaaatttcttgttcaggccggca
ttgtgaagaaggaacacatcaagattcatggtttctga

**SEQ ID NO 4:**

MSDLDIQIPTAFDPFAEANAGDSGAAAGSKDYVHVRIQQRNGRKSLTTVQGLKKEFSYNKIL
KDLKKEFCCNGTVVQDPELGQVIQLQGDQRKNVSNFLVQAGIVKKEHIKIHGF

**SEQ ID NO 5:**

gtatgtgcccttcggttgttcttggatttattgttctaggttgtgtagtacgggcgttgatg
ttaggaaaggggatctgtatctgtgatgattcctgttcttggatttgggatagaggggttct
tgatgttgcatgttatcggttcggtttgattagtagtatggttttcaatcgtctggagagct
ctatggaaatgaaatggtttagggtacggaatcttgcgattttgtgagtaccttttgtttga
ggtaaaatcagagcaccggtgattttgcttggtgtaataaaagtacggttgtttggtcctcg
attctggtagtgatgcttctcgatttgacgaagctatcctttgtttattccctattgaacaa
aaataatccaactttgaagacggtcccgttgatgagattgaatgattgattcttaagcctgt
ccaaaatttcgcagctggcttgtttagatacagtagtccccatcacgaaattcatggaaaca
gttataatcctcaggaacaggggattccctgttcttccgatttgctttagtcccagaatttt
ttttcccaaatatcttaaaaagtcactttctggttcagttcaatgaattgattgctacaaat
aatgcttttatagcgttatcctagctgtagttcagttaataggtaataccccctatagtttag
tcaggagaagaacttatccgatttctgatctccatttttaattatatgaaatgaactgtagc
ataagcagtattcatttggattatttttttattagctctcacccccttcattattctgagct
gaaagtctggcatgaactgtcctcaattttgttttcaaattcacatcgattatctatgcatt
atcctcttgtatctacctgtagaagtttctttttggttattccttgactgcttgattacaga
aagaaatttatgaagctgtaatcgggatagttatactgcttgttcttatgattcatttcctt
tgtgcag

**FIGURE 5 (continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1198985 A1
- EP 04102108 A
- US 60572141 B


### Non-patent literature cited in the description


- **ROSE ; LAST.** *Plant J.,* 1997, vol. 11, 455-464
- **CHAUBET-GIGOT et al.** *Plant Mol. Biol.,* 2001, vol. 45, 17-30
- **TANAKA et al.** *Nucleic Acids Res.,* 1990, vol. 18, 6767-6770
- **BRUCE ; QUAIL.** *Plant Cell,* 1990, vol. 2, 1081-1089
- **DEAN et al.** *Plant Cell,* 1989, vol. 1, 201-208
- **VAIN et al.** *Plant Cell Rep.,* 1996, vol. 15, 489-494
- **MCELROY et al.** *Plant Cell,* 1996, vol. 2, 163-171
- **SNOWDEN et al.** *Plant Mol. Biol.,* 1996, vol. 31, 689-692
- **RETHMEIER et al.** *Plant J.,* 1997, vol. 12, 895-899
- **KATO et al.** *Biosci. Biotechnol. Biochem.,* 1998, vol. 62, 151-153
- **LEON et al.** *Plant Physiol.,* 1991, vol. 95, 968-972
- **FU et al.** *Plant Cell,* 1995, vol. 7, 1387-1394
- **FU et al.** *Plant Cell,* 1995, vol. 7, 1395-1403
- **PLESSE et al.** *Plant Mol. Biol.,* 2001, vol. 45, 655-667
- **CALLIS et al.** *Genes Dev.,* 1987, vol. 1, 1183-1200
- **CLANCY et al.** *Plant Sci.,* 1994, vol. 98, 151-161
- **MASCARENHAS et al.** *Plant Mol. Biol.,* 1990, vol. 15, 913-920
- **ROSE.** *RNA,* 2002, vol. 8, 1444-1453
- **CLANCY ; HANNAH.** *Plant Physiol.,* 2002, vol. 130, 918-929
- **SINIBALDI ; METTLER.** Progress in Nucleic Acid Research and Molecular Biology. Academic Press, 1992, vol. 42, 229-257
- **LUEHRSEN ; WALBOT.** *Prog. Nucleic Acid Res. Mol. Biol.,* 1991, vol. 47, 149-193
- **MAAS et al.** *Plant Mol. Biol.,* 1991, vol. 16, 199-207
- **RETHMEIER et al.** *Plant J.,* 1998, vol. 13, 831-835
- **GALLIE ; YOUNG.** *Plant Physiol.,* 1994, vol. 106, 929-939
- **HENSGENS et al.** *Plant Mol. Biol.,* 1993, vol. 23, 643-669
- **DE PATER et al.** *Plant J.,* 1992, vol. 2, 837-844
- **BROWN.** *Plant Journal,* 1996, vol. 10, 771-780
- **SAMBROOK et al.** Molecular Cloning: a laboratory manual. Cold Spring Harbor Laboratory Press, 2001
- Current Protocols in Molecular Biology. John Wiley & Sons, 1989
- **NEEDLEMAN ; WUNSCH.** *J. Mol. Biol.,* 1970, vol. 48, 443-453
- **SMITH ; WATERMAN.** *Advances in Applied Mathematics,* 1981, vol. 2, 482-489
- **ALTSCHUL, S.F. ; GISH, W. ; MILLER, W. ; MYERS, E.W. ; LIPMAN, D.J.** *J. Mol. Biol.,* 1990, vol. 215, 403-410
- **W. R. PEARSON ; D. J. LIPMAN.** *Proc.Natl.Acad.Sci. USA,* 1988, vol. 85, 2444-2448
- **BORODOVSKY, M. ; MCLNINCH J.** GeneMark: Parallel Gene Recognition for both DNA Strands. *Computers & Chemistry,* 1993, vol. 17, 123-133, http://opal.biology.gatech.edu/genemark, http://www.ebi.ac.uk/genemark
- **A.L. DELCHER et al.** Improved microbial gene identification with GLIMMER. *Nucleic Acids Research,* 1999, vol. 27, 4636-4641, www.tigr.org/softiab
- **BURGE, C. ; KARLIN, S.** Prediction of complete gene structures in human genomic DNA. *J. Mol. Biol.,* 1997, vol. 268, 78-94, genes.mit.edu/GENSCAN.html
- **MILANESI L. et al.** GeneBuilder: interactive in silico prediction of genes structure. *Bioinformatics,* 1999, vol. 15 (7), 612-621, www.itba.mi.cnr.it/webgene

- **J. E. ALLEN et al.** Computational gene prediction using multiple sources of evidence. *Genome Research,* 2004, vol. 14 (1), 142-148, www.tigr.org/softlab
- **REESE MG et al.** Improved Splice Site Detection in Genie. *J Comp Biol,* 1997, vol. 4 (3), 311-23, www.fruitfly.org/seq_tools/splice.html
- **ROGOZIN I.B. ; L. MILANESI.** Analysis of donor splice signals in different organisms. *J. Mol. Evol.,* 1997, vol. 45, 50-59, www.itba.mi.cnr.it/webgene
- **S.M. HEBSGAARD et al.** Splice site prediction in Arabidopsis thaliana DNA by combining local and global sequence information. *Nucleic Acids Research,* 1996, vol. 24, 3439-3452, www.cbs.dtu.dk/services
- **PERTEA M. et al.** GeneSplicer: a new compulational method for splice site prediction. *Nucleic Acids Res.,* 01 March 2001, vol. 29 (5), 1185-90, www.tigr.org/softlab
- **KRENS, F.A. et al.** *Nature,* 1982, vol. 296, 72-74
- **NEGRUTIU I. et al.** *Plant Mol. Biol.,* 1987, vol. 8, 363-373
- **SHILLITO R.D. et al.** *Bio/Technol.,* 1985, vol. 3, 1099-1102
- **CROSSWAY A. et al.** *Mol. Gen. Genet.,* 1986, vol. 202, 179-185
- **KLEIN T.M. et al.** *Nature,* 1987, vol. 327, 70
- **ALDEMITA ; HODGES.** *Planta,* 1996, vol. 199, 612-617
- **CHAN et al.** *Plant Mol. Biol.,* 1993, vol. 22 (3), 491-506
- **HIEI et al.** *Plant J.,* 1994, vol. 6 (2), 271-282
- **ISHIDA et al.** *Nat. Biotechnol.,* June 1996, vol. 14 (6), 745-50
- **FRAME et al.** *Plant Physiol.,* May 2002, vol. 129 (1), 13-22
- **SAMBROOK.** Molecular Cloning: a laboratory manual. Cold Spring Harbor Laboratory Press, CSH, 2001
- **AUSUBEL et al.** Current Protocols in Molecular Biology. Current Protocols, vol. 1, 2
- **R.D.D. CROY.** Plant Molecular Biology Labfax. BIOS Scientific Publications Ltd (UK, 1993
- **BREYNE P et al.** *Plant Mol. Biol. Reporter,* 1993, vol. 11, 21-31

SEQUENCE LISTING

<110> CropDesign N.V.

<120> Method for increasing transgene expression

<130> CD-114-EP

<150> EP 04102108.0
<151> 2004-05-13

<150> US 60/572,141
<151> 2004-05-18

<160> 5

<170> PatentIn version 3.3

<210> 1
<211> 1112
<212> DNA
<213> Oryza sativa

<400> 1

```
ctagtagaag ccgagcgacc gccttcttcg atccatatct tccggtcgag ttcttggtcg      60
atctcttccc tcctccacct cctcctcaca gggtatgtgc ccttcggttg ttcttggatt     120
tattgttcta ggttgtgtag tacgggcgtt gatgttagga aaggggatct gtatctgtga     180
tgattcctgt tcttggattt gggatagagg ggttcttgat gttgcatgtt atcggttcgg     240
tttgattagt agtatggttt tcaatcgtct ggagagctct atggaaatga aatggtttag     300
ggtacggaat cttgcgattt tgtgagtacc ttttgtttga ggtaaaatca gagcaccggt     360
gattttgctt ggtgtaataa aagtacggtt gtttggtcct cgattctggt agtgatgctt     420
ctcgatttga cgaagctatc ctttgtttat tccctattga acaaaaataa tccaactttg     480
aagacggtcc cgttgatgag attgaatgat tgattcttaa gcctgtccaa aatttcgcag     540
ctggcttgtt tagatacagt agtccccatc acgaaattca tggaaacagt tataatcctc     600
aggaacaggg gattccctgt tcttccgatt tgctttagtc ccagaatttt ttttcccaaa     660
tatcttaaaa agtcactttc tggttcagtt caatgaattg attgctacaa ataatgcttt     720
tatagcgtta tcctagctgt agttcagtta ataggtaata cccctatagt ttagtcagga     780
gaagaactta tccgatttct gatctccatt tttaattata tgaaatgaac tgtagcataa     840
gcagtattca tttggattat ttttttatt agctctcacc ccttcattat tctgagctga      900
aagtctggca tgaactgtcc tcaattttgt tttcaaattc acatcgatta tctatgcatt     960
atcctcttgt atctacctgt agaagtttct ttttggttat tccttgactg cttgattaca    1020
gaaagaaatt tatgaagctg taatcgggat agttatactg cttgttctta tgattcattt    1080
cctttgtgca gttcttggtg tagcttgcca ct                                  1112
```

<210> 2
<211> 1176
<212> DNA
<213> Oryza sativa

<400> 2

```
taggaggcat ccaagccaag aagagggaga gcaccaagga cacgcgacta gcagaagccg      60
agcgaccgcc ttcttcgatc catatcttcc ggtcgagttc ttggtcgatc tcttccctcc     120
tccacctcct cctcacaggg tatgtgccct tcggttgttc ttggatttat tgttctaggt     180
tgtgtagtac gggcgttgat gttaggaaag gggatctgta tctgtgatga ttcctgttct     240
tggatttggg atagaggggt tcttgatgtt gcatgttatc ggttcggttt gattagtagt     300
atggttttca atcgtctgga gagctctatg gaaatgaaat ggtttagggt acggaatctt     360
gcgattttgt gagtaccttt tgtttgaggt aaaatcagag caccggtgat tttgcttggt     420
gtaataaaag tacggttgtt tggtcctcga ttctggtagt gatgcttctc gatttgacga     480
agctatcctt tgtttattcc ctattgaaca aaaataatcc aactttgaag acggtcccgt     540

tgatgagatt gaatgattga ttcttaagcc tgtccaaaat ttcgcagctg gcttgtttag     600
atacagtagt ccccatcacg aaattcatgg aaacagttat aatcctcagg aacaggggat     660
tccctgttct tccgatttgc tttagtccca gaattttttt tcccaaatat cttaaaaagt     720
cactttctgg ttcagttcaa tgaattgatt gctacaaata atgcttttat agcgttatcc     780
tagctgtagt tcagttaata ggtaataccc ctatagttta gtcaggagaa gaacttatcc     840
gatttctgat ctccattttt aattatatga aatgaactgt agcataagca gtattcattt     900
ggattatttt ttttattagc tctcacccct tcattattct gagctgaaag tctggcatga     960
actgtcctca attttgtttt caaattcaca tcgattatct atcgattatc ctcttgtatc    1020
tacctgtaga agtttctttt tggttattcc ttgactgctt gattacagaa agaaatttat    1080
gaagctgtaa tcgggatagt tatactgctt gttcttatga ttcatttcct ttgtgcagtt    1140
cttggtgtag cttgccactt tcaccagcaa agtttc                              1176
```

<210> 3
<211> 348
<212> DNA
<213> Oryza sativa

<400> 3

```
atgtctgatc tcgacattca gatcccaact gccttcgatc cctttgctga ggccaatgct     60
ggagactctg gtgcggctgc aggatcaaag gactacgttc atgtacgcat ccagcagcgt    120
aatggccgta agagcctgac cactgtccag ggattgaaga aggaattcag ctacaacaag    180
atccttaaag atctcaagaa agagttttgc tgcaatggta ctgttgtcca ggacccagag    240
cttggccagg tcattcaact tcagggtgat cagaggaaga acgtatcaaa ttttcttgtt    300
caggccggca ttgtgaagaa ggaacacatc aagattcatg gtttctga                 348
```

<210> 4
<211> 115
<212> PRT
<213> Oryza sativa

<400> 4

```
Met Ser Asp Leu Asp Ile Gln Ile Pro Thr Ala Phe Asp Pro Phe Ala
1               5                   10                  15

Glu Ala Asn Ala Gly Asp Ser Gly Ala Ala Ala Gly Ser Lys Asp Tyr
            20                  25                  30

Val His Val Arg Ile Gln Gln Arg Asn Gly Arg Lys Ser Leu Thr Thr
        35                  40                  45

Val Gln Gly Leu Lys Lys Glu Phe Ser Tyr Asn Lys Ile Leu Lys Asp
    50                  55                  60

Leu Lys Lys Glu Phe Cys Cys Asn Gly Thr Val Val Gln Asp Pro Glu
65                  70                  75                  80

Leu Gly Gln Val Ile Gln Leu Gln Gly Asp Gln Arg Lys Asn Val Ser
                85                  90                  95

Asn Phe Leu Val Gln Ala Gly Ile Val Lys Lys Glu His Ile Lys Ile
            100                 105                 110

His Gly Phe
        115
```

<210> 5
<211> 999
<212> DNA
<213> Oryza sativa

<400> 5

```
gtatgtgccc ttcggttgtt cttggattta ttgttctagg ttgtgtagta cgggcgttga     60
tgttaggaaa ggggatctgt atctgtgatg attcctgttc ttggatttgg gatagagggg    120
ttcttgatgt tgcatgttat cggttcggtt tgattagtag tatggttttc aatcgtctgg    180
agagctctat ggaaatgaaa tggtttaggg tacggaatct tgcgattttg tgagtacctt    240
ttgtttgagg taaaatcaga gcaccggtga ttttgcttgg tgtaataaaa gtacggttgt    300
ttggtcctcg attctggtag tgatgcttct cgatttgacg aagctatcct ttgtttattc    360
cctattgaac aaaaataatc caactttgaa gacggtcccg ttgatgagat tgaatgattg    420
attcttaagc ctgtccaaaa tttcgcagct ggcttgttta gatacagtag tccccatcac    480
gaaattcatg gaaacagtta taatcctcag gaacagggga ttccctgttc ttccgatttg    540
ctttagtccc agaatttttt ttcccaaata tcttaaaaag tcactttctg gttcagttca    600
atgaattgat tgctacaaat aatgctttta tagcgttatc ctagctgtag ttcagttaat    660
aggtaatacc cctatagttt agtcaggaga agaacttatc cgatttctga tctccatttt    720
taattatatg aaatgaactg tagcataagc agtattcatt tggattattt tttttattag    780
ctctcacccc ttcattattc tgagctgaaa gtctggcatg aactgtcctc aattttgttt    840
tcaaattcac atcgattatc tatgcattat cctcttgtat ctacctgtag aagtttcttt    900
ttggttattc cttgactgct tgattacaga aagaaattta tgaagctgta atcgggatag    960
ttatactgct tgttcttatg attcatttcc tttgtgcag                           999
```